# EUROPÄISCHE PATENTSCHRIFT

(11) **EP 3 313 516 B1**
(45) Veröffentlichungstag und Bekanntmachung des Hinweises auf die Patenterteilung: **09.04.2025**
(21) Anmeldenummer: 16741986.0
(22) Anmeldetag: 29.06.2016
(51) Int. Cl.: A61N 7/00

(54) **PATIENTENSPEZIFISCHE FUNKTIONELLE CRANIOPLASTIE MIT INTEGRIERTER ULTRASCHALLSENDER-ANORDNUNG FÜR NEUROMODULATION UND BLUTHIRNSCHRANKENÖFFNUNG**
PATIENT-SPECIFIC FUNCTIONAL CRANIOPLASTY WITH INTEGRATED ULTRASONIC TRANSMITTER ARRANGEMENT FOR NEUROMODULATION AND OPENING OF THE BLOOD BRAIN BARRIER
CRANIOPLASTIE FONCTIONNELLE SPÉCIFIQUE AU PATIENT AU MOYEN D'UN DISPOSITIF D'ÉMETTEURS D'ULTRASONS INTÉGRÉ POUR LA NEUROMODULATION ET L'OUVERTURE DE LA BARRIÈRE HÉMATO-ENCÉPHALIQUE

(30) Priorität: 29.06.2015 CH 9632020
(43) Veröffentlichungstag der Anmeldung: 02.05.2018
(73) Patentinhaber: Bauer, Ronald, 9053 Teufen (CH); Schindel, Ralf, 9011 St. Gallen (CH); Dumont, Erik, 33600 (FR); Martin-Fiori, Ernst, 8032 Zürich (CH); Werner, Beat, 8032 Zürich (CH)
(72) Erfinder: Bauer, Ronald, 9053 Teufen (CH); Schindel, Ralf, 9011 St. Gallen (CH); Dumont, Erik, 33600 (FR); Martin-Fiori, Ernst, 8032 Zürich (CH); Werner, Beat, 8032 Zürich (CH)
(74) Vertreter: Stolmár & Partner Patentanwälte PartG mbB
(86) Internationale Anmeldenummer: PCT/IB2016/000925
(87) Internationale Veröffentlichungsnummer: WO 2017/001911

(56) Entgegenhaltungen:
- EP-B1- 3 419 548
- WO-A1-2011/101731
- WO-A1-2013/017778
- WO-A1-2014/031142
- WO-A2-2016/097867
- WO-A2-2016/097867
- CN-A- 104 545 919
- US-A1- 2011 160 621
- US-A1- 2011 160 621
- US-A1- 2011 319 927
- US-A1- 2013 204 316
- US-A1- 2013 204 316
- US-A1- 2014 058 292
- US-A1- 2014 249 454

## Beschreibung

Die Erfindung bezieht sich auf eine Ultraschallsender-Anordnung .

Die Verwendung mechanischer Wellen, um in einem bestimmten lokalen Bereich eines menschlichen Körpers durch gezieltes Eintragen mechanischer Energie einen gewünschten lokalen Effekt hervorzurufen, ist an sich bekannt.

Fokussierte Ultraschallwellen können z.B. zur Zertrümmerung von Nierensteinen eingesetzt werden. Die eingetragene mechanische Energie wird in den Nierensteinen absorbiert und führt zu deren Zerkleinerung. Fokussierte Ultraschallwellen können auch in weichem Körpergewebe verwendet werden, um krankes oder abnormales Gewebe, wie z.B. einen Tumor oder einen Thrombus, welcher zu Funktionsstörungen im Organismus führt, ggfs. zusammen mit einer chemischen Behandlung zu beseitigen (Tumorablation, Thrombolyse mit Ultraschallunterstützung).

Auch für Behandlungen des zentralen Nervensystems, insbesondere des Gehirns (siehe beispielsweise US 2013/204316 A1, die eine Vorrichtung zur Behandlung einer Hirnschädigung, insbesondere eines Gehirntumors offenbart), können fokussierter Ultraschall mit hoher Intensität (HIFU/high intensity focused ultrasound) oder fokussierter Ultraschall mit niedriger Intensität (LIFU/Low intensity focused ultrasound) verwendet werden. Der lokale Bereich, in welchem dabei Ultraschallenergie eingetragen wird, kann mittels Methoden der Magnetresonanz während des Ultraschalleingriffs überwacht werden, um eine gezielte Steuerung des Ultraschalleingriffs zu ermöglichen (transcranielle Magnetresonanz-gesteuerte fokussierte Ultraschall-Chirurgie, tcMRgFUS). Gegenüber anderen chirurgischen, chemischen oder physikalischen Behandlungsverfahren, die oftmals negative Auswirkungen auf den Patienten haben (Blutungs- und Infektionsrisiko, chemische Toxizität, radioaktive Strahlung, Dosisakkumulation, etc.) stellt eine derartige tcMRgFUS-Behandlung einen minimalinvasiven Eingriff dar, der einen sofortigen Effekt zeigt, wiederholbar ist und in der Regel ambulant oder kurzzeitig stationär durchgeführt werden kann.

Für Eingriffe im Gehirn hat sich als minimalinvasives Verfahren tcMRgFUS (HIFU oder LIFU) als vielversprechend erwiesen, der von mehreren ausserhalb des Schädels angeordneten Ultraschallsendern durch den Schädelknochen und anschliessend durch andere Hirnbereiche hindurch zu dem zu behandelnden Hirnbereich ("Zielbereich") ausgesendet wird, wo alle Ultraschallwellen in einem definierten Focus zusammentreffen.

Bei diesem Verfahren tritt jedoch regelmässig die Schwierigkeit auf, dass die von aussen auf den Zielbereich im Gehirn gerichteten fokussierten Ultraschallwellen beim Durchqueren des Schädelknochens je nach dessen örtlicher Dicke und Dichte sowie je nach dem Winkel zwischen der Ausbreitungsrichtung (Strahlrichtung) der Ultraschallwellen unterschiedlich stark verzögert, abgelenkt und abgeschwächt werden (Laufzeitunterschiede, Brechung bzw. Absorption durch Wand des Schädelknochens). Durchschnittlich gehen z.B. bei 650kHz 90% der ausgesendeten Ultraschallenergie durch Absorption im Bereich des Schädelknochens und der Kopfhaut verloren. Die Wirkung der verbleibenden Energie für die intracranielle Fokussierung kann zusätzlich durch folgende Probleme reduziert werden:
- konvergieren der eingestrahlten Ultraschallwellen an verschiedenen Orten (DeFokussierung, sekundäre Fokusbildung)
- Eintreffen der eingestrahlten Ultraschallenergie zu unterschiedlichen Zeitpunkten an ihrem jeweiligen Fokus (Laufzeitverschiebung).
- auftretende Reflexionen

Die Gesamtheit dieser Phänomene trägt zu einer signifikanten Schwächung der Wirkung der Ultraschallwellen im Zielbereich bei.

Um hier Abhilfe zu schaffen, kann der Schädelknochen mittels Computertomografie analysiert werden, wodurch man den räumlichen Verlauf der inneren und äusseren Grenzfläche des Schädelknochens die innere Struktur und die Knochendichte erhält. Aufgrund dieser Information können die räumliche Ausrichtung (Grad der Fokussierung) der jeweiligen Ultraschallsender und/oder deren Ansteuerungssignal (Impuls-Phasenverschiebung) verändert werden, um eine Kompensation der örtlichen und zeitlichen Fehler der Ultraschallwellen im Zielbereich zu erzielen. Diese Kompensation des Einflusses des Schädelknochens auf den fokussierten Ultraschall ist recht kompliziert. Die folgenden Dokumente gelten als relevanter Stand der Technik: US2014/249454), WO2014/031142, WO2016/097867, US2011/160621, US 2011/319927, CN104545919) und WO2011101731.

Der Erfindung liegt die Aufgabe zugrunde, die Schwierigkeiten der hier beschriebenen transcraniellen Magnetresonanz-gesteuerten fokussierten Ultraschall-Chirurgie (tcMRgFUS) zu minimieren oder ganz zu beseitigen.

Zur Lösung dieser Aufgabe stellt die Erfindung eine geeignete Ultraschallsender-Anordnung bereit mit einer an der Anordnung verteilt angeordneten Vielzahl von Ultraschallsendern, deren ausstrahlbare Ultraschallwellen zu einem gemeinsamen Fokus hin fokussiert sind oder fokussiert werden können, wobei die Anordnung als Schädelknochen-Implantat ausgebildet ist, welches als Ersatz für einen Teil des Schädelknochens einer Person in deren Schädelknochen implantierbar ist, und wobei im implantierten Zustand der gemeinsame Fokus der Anordnung in einer definierten Hirnregion liegt oder dorthin gesteuert werden kann, dadurch gekennzeichnet, dass die Anordnung ein individualisiertes rekonstruiertes Implantat ist, welches auf Basis von dreidimensionalen Bilddaten des Kopfes, d.h. von Gehirn und Schädelknochen, einer Person hergestellt wurde.

Die erfindungsgemässe Ultraschallsender-Anordnung kann dem Patienten durch einen einmaligen chirurgischen Eingriff in seinen Schädelknochen bzw. als Ersatz für einen Teil seines Schädelknochens implantiert werden. Dieses Implantat ist für LIFU-FUS geeignet. LIFU-FUS-Eingriffe dienen vorwiegend einer regelmässigen bis quasi-permanenten nichtinvasiven Beeinflussung ("Neuro-Modulation") eines möglichst kleinen Hirnbereiches, der für Fehlfunktionen des zentralen Nervensystems verantwortlich ist. Diese Eingriffe sind typischerweise reversibel, d.h. man kann einen gewissen lokalen Hirnbereich, der für eine Fehlfunktion des zentralen Nervensystems hauptsächlich verantwortlich ist, vorrübergehend ausschalten oder stimulieren. "Vorrübergehend" kann dabei je nach neuroanatomischem Zielbereich und je nach Parameterauswahl und Dauer des FUS-Eingriffs einige Minuten, einige Stunden, einige Tage, einige Wochen, einige Monate oder noch länger bedeuten. Die Ultraschallsender sind durch deren Anordnung im Implantat stets am richtigen Ort bezüglich des gewissen lokalen Hirnbereichs parat und können bei einer notwendigen Wiederholung des LIFU- Eingriffs schnell aktiviert werden. Damit stellt die Erfindung eine Alternative zum Hirnschrittmacher (Implantation von Elektroden ins Gehirn) dar.

Vorzugsweise kann das Schädelknochenimplantat als Single- oder Multipleelement (Mehrfachimplantat) angewandt werden.

Vorzugsweise ist das Schädelknochen-Implantat als Kalotte ausgebildet und die Ultraschall-Ausstrahlbereiche der Ultraschallsender sind im implantierten Zustand an der dem Gehirn zugewandten Oberfläche der Kalotte angeordnet.

Dies hat den Vorteil, dass die ins Innere des Gehirns ausgestrahlten Ultraschallwellen keinen Bereich des Schädelknochens durchlaufen müssen, sondern sich lediglich durch die Dura mater und das Hirnparenchym bis zu dem Zielbereich im Gehirn ausbreiten müssen, wodurch es keine signifikanten Unterschiede in der Ausbreitungsgeschwindigkeit der Ultraschallwellen (Phasenkohärenz) und somit praktisch keinerlei Ablenkung der zu dem Zielbereich hin fokussierten Ultraschallwellen gibt. Die Ausrichtungen und/oder die Phasen des Ansteuerungssignals der einzelnen Ultraschallsender gestalten sich daher viel einfacher als bei einer Ultraschallsender-Anordnung ausserhalb des Schädelknochens.

Gemäss einer ersten Ausgestaltung ist die Kalotte dem entnommenen Teil des Schädelknochens nachempfunden.

Dadurch wird neben dem rein kosmetischen Aspekt gewährleistet, dass der Patient sein Implantat als möglichst wenig störend empfindet.

Gemäss einer zweiten Ausgestaltung ist die Kalotte ein Kugelschalen-Segment, an welchem die Ultraschallsender verteilt, insbesondere gleichmässig oder pseudo-randomisiert verteilt, angeordnet sind, und ist der gemeinsame natürliche Fokus der Ultraschallsender der Kugelmittelpunkt des Kugelschalen-Segments.

Dies ermöglicht die Verwendung einer sphärischen Ultraschallsender-Anordnung mit einer phasengesteuerten Abstrahl-Charakteristik (spherical ultrasound phased array, vgl. Antennendiagramm einer phasengesteuerten Gruppenantenne), wodurch sich das Intensitätsmaximum bzw. der Fokus des Ultraschall-Wellenfeldes örtlich verschieben lässt.

Die Ausgestaltung der Kalotte kann auch eine Zwischenform zwischen der ersten Ausgestaltung und der zweiten Ausgestaltung sein, so dass man einen Kompromiss zwischen den Vorteilen der beiden Ausgestaltungen erzielt.

Vorzugsweise ist der Aussenrand der Kalotte zum Innenrand der durch Entnahme des Teils des Schädelknochens entstanden Schädelöffnung komplementär.

Dies erleichtert es dem Chirurgen, dem Patienten durch den einmaligen chirurgischen Eingriff in dessen Schädelknochen bzw. als Ersatz für einen oder mehrere Teile seines Schädelknochens die erfindungsgemässe Ultraschallsender-Anordnung in Kalottenform zu implantieren.

Bei einer besonders bevorzugten Ausführung ist die Anordnung ein individualisiertes rekonstruiertes Implantat, welches auf Basis von dreidimensionalen Bilddaten des Kopfes, d.h. von Gehirn und Schädelknochen, einer Person hergestellt wurde, wobei vorzugsweise das individualisierte rekonstruierte Implantat mittels eines additiven Verfahrens hergestellt wurde.

Der Ultraschallsender-Anordnung kann eine Batterie, insbesondere eine wieder aufladbare Batterie, zugeordnet sein. Diese kann in unmittelbarer Nähe an der Ultraschallsender-Anordnung implantiert sein. Der Ultraschallsender-Anordnung kann auch ein Elektrizitäts-Generator, insbesondere ein thermoelektrischer Generator oder eine Blutzucker-Brennstoffzelle, zugeordnet sein. Der Elektrizitäts-Generator kann in unmittelbarer Nähe an der Ultraschallsender-Anordnung implantiert sein. Zweckmässigerweise wird der Elektrizitäts-Generator zum Laden der wieder aufladbaren Batterie verwendet. Vorzugsweise wird ein thermoelektrischer Generator zusammen mit der Ultraschallsender-Anordnung verwendet. Der thermoelektrische Generator wird vorzugsweise im Schädelknochen implantiert und nutzt den Temperaturgradienten zwischen der inneren Oberfläche und der äusseren Oberfläche des Schädelknochens. Zur Lösung der weiter oben erwähnten Aufgabe stellt die Offenlegung auch ein Verfahren bereit zur Behandlung einer

Person, welche an Störungen des zentralen Nervensystems leidet, wobei das Behandlungsverfahren die folgenden Schritte aufweist:
- Identifizieren einer definierten Hirnregion der Person zur Beeinflussung der zugrunde liegenden Pathologie;
- Implantieren einer oder mehrerer Ultraschallsender-Anordnungen, wie sie in einem der vorhergehenden Absätze definiert ist, in den Schädelknochen der Person; und
- Aktivieren von Ultraschallsendern der Ultraschallsender-Anordnung, um der definierten Hirnregion gezielt Ultraschallenergie zuzuführen.

Das Aktivieren der Ultraschallsender kann derart erfolgen, dass die Energie der ausgestrahlten Ultraschallwellen eine reversible Beeinflussung der definierten Hirnregion bewirkt (LIFU). In diesem Niedrig-Energie-Modus wird eine Neuro-Modulation oder, in Kombination mit Ultraschallkontrastmittel, eine Öffnung der Blut-Hirn-Schranke in dem Zielbereich bewirkt. Vorzugsweise wird dabei das Öffnen der Blut-Hirn-Schranke mittels LIFU-FUS bewirkt, um dem Gehirn bzw. dem zentralen Nervensystem sehr spezifisch wirkende Pharmazeutika in Form von Makromolekülen zuzuführen. Die Energiezufuhr kann dabei einerseits auf direktem Weg mittels implantiertem Impulsgenerator und zuführenden Elektroden an die Transducer-Elemente oder andererseits telemetrisch transkutan erfolgen.

Zur Lösung der weiter oben erwähnten Aufgabe stellt die Erfindung auch noch ein Verfahren unter Verwendung einer wie vorstehend beschriebenen Ultraschallsender-Anordnung bereit zum Herstellen eines Übungsmodells für die Behandlung einer Person, welche an Störungen des zentralen Nervensystems leidet, wobei das Verfahren die folgenden Schritte aufweist:
- Identifizieren einer definierten Hirnregion der Person zur Beeinflussung der zugrunde liegenden Pathologie;
- Erfassen dreidimensionaler Bilddaten des Kopfes, d.h. von Gehirn und Schädel, der Person;
- Herstellen eines individualisierten rekonstruierten dreidimensionalen Kopfmodells auf Basis von dreidimensionalen Bilddaten des Kopfes; und
- Anordnen einer Vielzahl von Ultraschallsendern in einem Schädelknochenbereich des Kopfmodells, wobei die ausstrahlbaren Ultraschallwellen zu der definierten Hirnregion des Kopfmodells als gemeinsamem Fokus hin fokussiert werden.

Dieses Übungsmodell kann verwendet werden, um patienten- und krankheitsspezifische Versuche mittels HIFU-FUS und mittels LIFU-FUS an dem Übungsmodell durchzuführen.

Vorzugsweise enthält das Verfahren zum Herstellen eines Übungsmodells einen weiteren Schritt zum Definieren eines ausgeschnittenen Schädelknochenbereichs mit den darin angeordneten Ultraschallsendern als Modell für ein Implantat, wie es in einem der vorhergehenden Absätze definiert ist, sowie ein Modell für eine Schneidlehre zur Herstellung einer Schädelöffnung während eines chirurgischen Eingriffs beim Implantieren des erfindungsgemässen Implantats.

Weitere Vorteile, Merkmale und Anwendungsmöglichkeiten der Erfindung ergeben sich aus der folgenden, nicht einschränkend aufzufassenden Beschreibung eines Ausführungsbeispiels der erfindungsgemässen Ultraschallsender-Anordnung anhand der Zeichnung, wobei
Fig. 1 eine schematische Darstellung einer Ultraschallsender-Anordnung für fokussierten Ultraschall in einer ersten Anwendungsumgebung ist;
Fig. 2 eine schematische Darstellung einer Ultraschallsender-Anordnung für fokussierten Ultraschall in einer zweiten Anwendungsumgebung ist;
Fig. 3 eine schematische Darstellung einer Ultraschallsender-Anordnung für fokussierten Ultraschall in einer dritten Anwendungsumgebung ist;
Fig. 4A eine Schnittansicht eines Schädels ist, der einer Behandlung mittels einer Ultraschallsender-Anordnung ausgesetzt ist; und
Fig. 4B eine Perspektivansicht eines Schädelknochens mit einer implantierten Ultraschallsender-Anordnung gemäss der Erfindung ist.

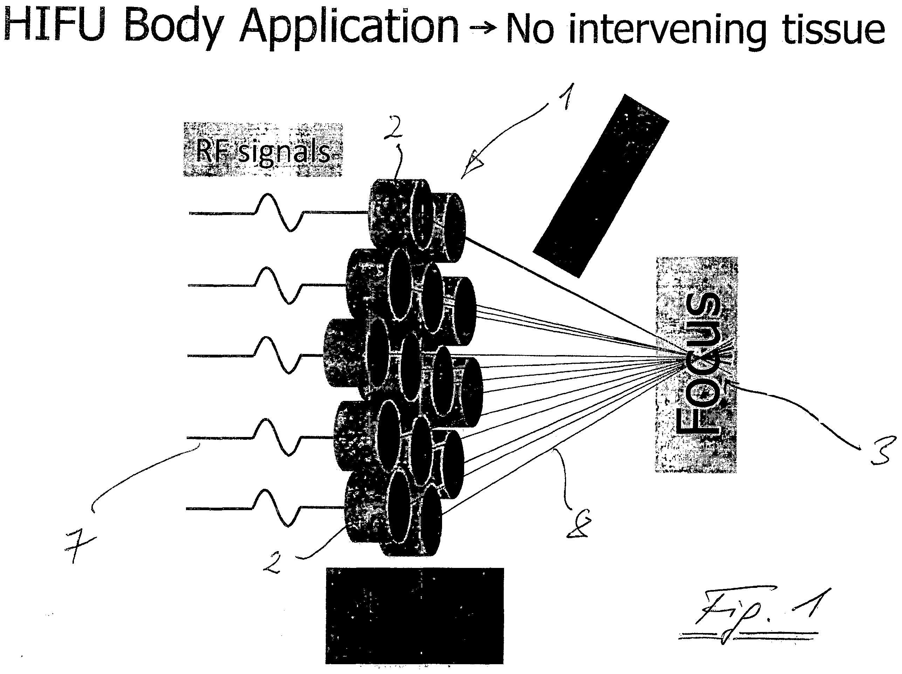

In Fig. 1 ist eine schematische Darstellung einer Ultraschallsender-Anordnung 1 für fokussierten Ultraschall in einer ersten Anwendungsumgebung gezeigt. Diese Ultraschallsender-Anordnung 1 enthält eine Vielzahl von Ultraschallsendern 2, die jeweils mittels eines elektrischen Signals 7 angesteuert werden und jeweils eine fokussierte Ultraschallwelle aussenden. Anstelle der Wellenfronten sind die Ultraschallwellen mittels eines Strahls 8 dargestellt, der orthogonal zu den jeweiligen Wellenfronten ausgerichtet ist. Jede der Wellenfronten bzw. jeder der Strahlen 8 konvergiert in einem gemeinsamen Fokus 3. Im Fokus 3 addieren sich die Intensitäten sämtlicher Ultraschallwellen. Die gebündelte Ultraschallenergie aller Ultraschallwellen wird im Fokus 3 in das dortige Gewebe (z.B. Knochen, Bindegewebe, Muskeln, Nerven, etc.) oder in einen Fremdkörper (z.B. Nierenstein, Blutgerinnsel, etc.) eingetragen und bewirkt dort mehr oder weniger starke Veränderungen durch direkte mechanische oder indirekte thermische Einwirkung auf das Gewebe oder den Fremdkörper.

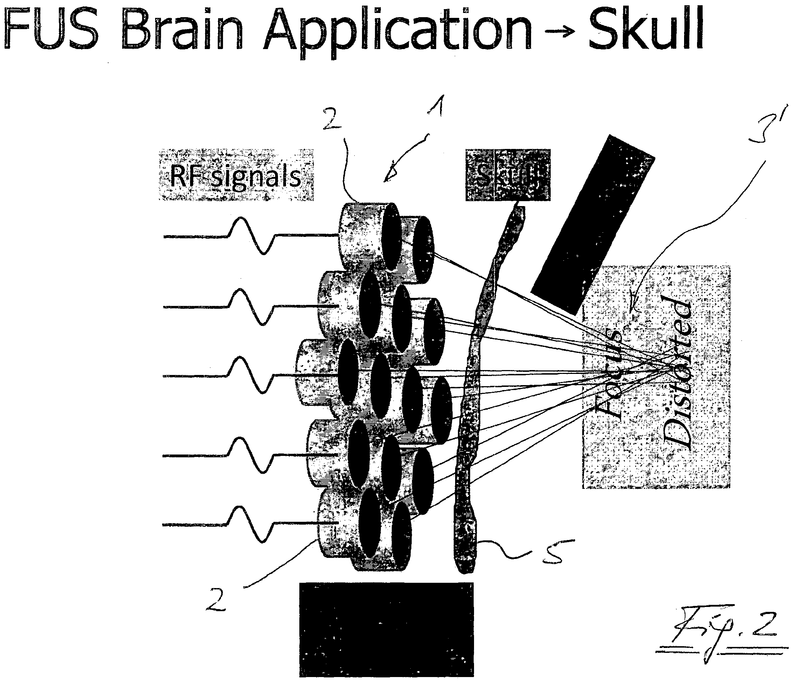

In Fig. 2 ist eine schematische Darstellung einer Ultraschallsender-Anordnung 1 für fokussierten Ultraschall in einer zweiten Anwendungsumgebung gezeigt. Diese in Fig. 2 gezeigte Darstellung unterscheidet sich von der in Fig. 1 gezeigten Darstellung dadurch, dass zwischen der Ultraschallsender-Anordnung 1 und dem Fokus 3 ein Schädelknochen 5 angeordnet ist. Diese Anwendungsumgebung dient für Eingriffe im Gehirn mittels fokussierten Ultraschalls (HIFU oder LIFU) als minimalintensives Verfahren, wobei der von den ausserhalb des Schädels angeordneten Ultraschallsendern 2 ausgesendete Ultraschall durch den Schädelknochen 5 und anschliessend durch andere Hirnbereiche hindurch zu dem zu behandelnden Hirnbereich ("Zielbereich") im Fokus 3 ausgesendet wird. Man möchte, dass dort alle fokussierten Ultraschallwellen zusammentreffen (transkranielle MRgFUS). Die gemäss Fig. 1 angeordneten Ultraschallsender 2 richten fokussierte Ultraschallwellen von aussen auf den Zielbereich im Gehirn. Beim Durchqueren des Schädelknochens 5 werden die Ultraschallwellen im Vergleich zu ihrer in Fig. 1 dargestellten Ausbreitung je nach örtlicher Dicke und Dichte des Schädelknochens 5 sowie je nach Winkel zwischen der Ausbreitungsrichtung der Ultraschallwellen und der Ausrichtung des Schädelknochens 5 unterschiedlich stark verzögert, abgelenkt und abgeschwächt. Die von den Ultraschallsendern 2 ausserhalb des Schädels eingestrahlten Ultraschallwellen konvergieren daher an verschiedenen Orten und im Falle gepulster Ultraschallwellen auch zu unterschiedlichen Zeitpunkten an ihrem jeweiligen Fokus. Insgesamt führt dies zu einer Defokussierung 3' und dadurch zu einer massiven Schwächung der Wirkung der Ultraschallwellen im Zielbereich im Vergleich zu der gemäss Fig. 1 erzielten Wirkung.

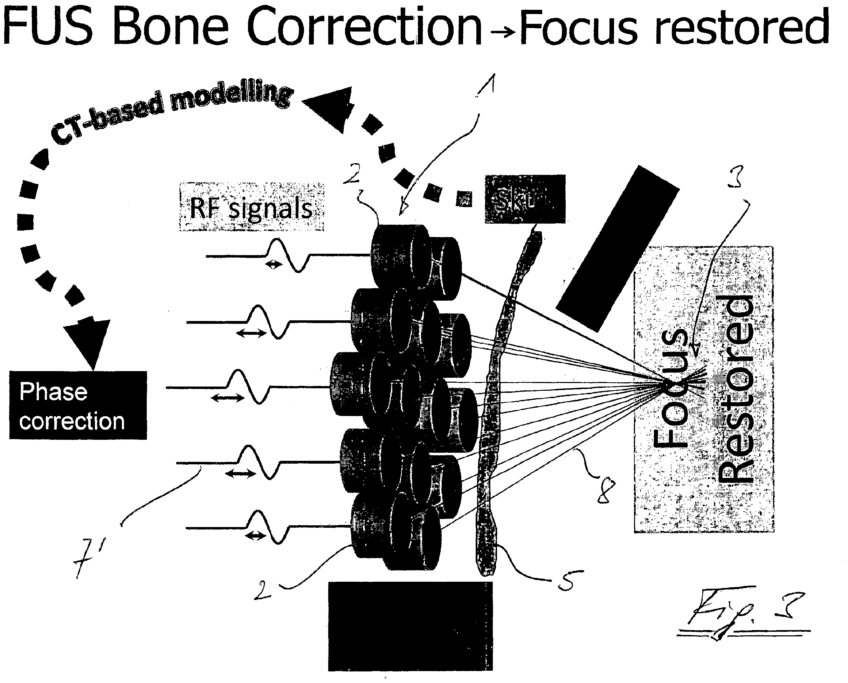

In Fig. 3 ist eine schematische Darstellung einer Ultraschallsender-Anordnung 1 für fokussierten Ultraschall in einer dritten Anwendungsumgebung gezeigt. Diese in Fig. 3 gezeigte Darstellung unterscheidet sich von der in Fig. 2 gezeigten Darstellung dadurch, dass die einzelnen Ultraschallsender 2 der Ultraschallsender-Anordnung 1 jeweils mittels eines phasen-korrigierten elektrischen Signals 7' angesteuert werden und jeweils eine fokussierte Ultraschallwelle mit einer korrigierten Wellenfront bzw. Strahlrichtung aussenden, was zur Folge hat, dass nun wieder wie in Fig. 1 alle Wellenfronten bzw. jeder der Strahlen 8 in einem gemeinsamen Fokus 3 konvergieren und sich im Fokus 3 die Intensitäten sämtlicher Ultraschallwellen addieren.

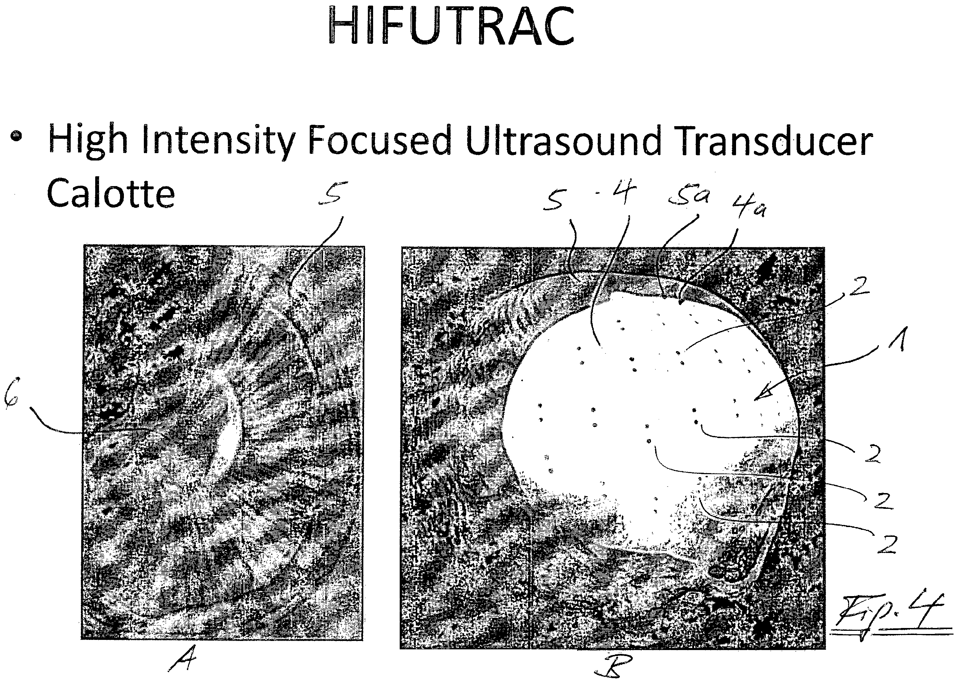

In Fig. 4A ist eine MRI-Schnittansicht eines Schädels gezeigt, der einer Behandlung mittels einer Ultraschallsender-Anordnung 1 ausgesetzt ist. Man erkennt den das Gehirn umgebenden Schädelknochen 5 sowie einen Zielbereich 6 im Gehirn, der dem Fokus 3 (siehe Fig. 1, Fig. 3) der Ultraschallwellen entspricht.

In Fig. 4B ist eine Perspektivansicht eines Schädelknochens 5 mit einer implantierten Ultraschallsender-Anordnung 1 gemäss der Erfindung gezeigt. Die erfindungsgemässe Ultraschallsender-Anordnung 1 hat die Form einer Kalotte 4, in welcher eine Vielzahl von Ultraschallsendern 2 angeordnet ist. Der Aussenrand 4a der Kalotte 4 ist zum Innenrand 5a der durch Entnahme des Teils des Schädelknochens 5 entstanden Schädelöffnung komplementär. Dies erleichtert das Implantieren der Kalotte 4 in dem Schädelknochen 5 bzw. als Ersatz für einen Teil des Schädelknochens 5. Die Kalotte 4 ist ein individualisiertes rekonstruiertes Implantat, welches auf Basis von dreidimensionalen Bilddaten des Kopfes, d.h. von Gehirn und Schädelknochen, des Patienten mittels eines additiven Verfahrens hergestellt wurde.

## Patentansprüche

1. Ultraschallsender-Anordnung (1) mit einer an der Anordnung verteilt angeordneten Vielzahl von Ultraschallsendern (2), deren ausstrahlbare Ultraschallwellen zu einem gemeinsamen Fokus (3) hin fokussiert sind oder fokussiert werden können, wobei die Anordnung (1) als Schädelknochen-Implantat (4) ausgebildet ist, welches als Ersatz für einen Teil des Schädelknochens (5) einer Person in deren Schädelknochen (5) implantierbar ist, und wobei im implantierten Zustand der gemeinsame Fokus(3) der Anordnung (1) in einer definierten Hirnregion (6) liegt oder dorthin gesteuert werden kann, **dadurch gekennzeichnet, dass** die Anordnung ein individualisiertes rekonstruiertes Implantat ist, welches auf Basis von dreidimensionalen Bilddaten des Kopfes, d.h. von Gehirn und Schädelknochen, einer Person hergestellt wurde.

2. Ultraschallsender-Anordnung (1) nach Anspruch 1 **dadurch gekennzeichnet, dass** das Schädelknochen-Implantat (4) als Kalotte ausgebildet ist und die Ultraschall-Ausstrahlbereiche der Ultraschallsender (2) an der im implantierten Zustand dem Gehirn zugewandten Oberfläche der Kalotte (4) angeordnet sind.

3. Ultraschallsender-Anordnung (1) nach Anspruch 2 **dadurch gekennzeichnet,**
**dass** die Kalotte (4) dem entnommenen Teil des Schädelknochens (5) nachempfunden ist.

4. Ultraschallsender-Anordnung (1) nach Anspruch 2 **dadurch gekennzeichnet,**
**dass** die Kalotte (4) ein Kugelschalen-Segment ist, an welchem die Ultraschallsender (2) verteilt angeordnet sind, und der gemeinsame Fokus der Ultraschallsender (2) der Kugelmittelpunkt des Kugelschalen-Segments ist.

5. Ultraschallsender-Anordnung (1) nach Anspruch 4, **dadurch gekennzeichnet,**
**dass** der Aussenrand (4a) der Kalotte (4) zum Innenrand (5a) der durch Entnahme des Teils des Schädelknochens (5) entstanden Schädelöffnung komplementär ist.

6. Ultraschallsender-Anordnung nach einem der Ansprüche 1 bis 5 **dadurch**
**gekennzeichnet, dass** das individualisierte rekonstruierte Implantat mittels eines additiven Verfahrens hergestellt wurde.

7. Ultraschallsender-Anordnung nach einem der vorhergehenden Ansprüche, **dadurch gekennzeichnet, dass** ihr eine Batterie, insbesondere eine wieder aufladbare Batterie, zugeordnet ist.

8. Ultraschallsender-Anordnung nach einem der vorhergehenden Ansprüche, **dadurch gekennzeichnet, dass** ihr ein Generator für elektrische Energie, insbesondere ein thermoelektrischer Generator oder eine Blutzucker-Brennstoffzelle, zugeordnet ist.

9. Verfahren zum Herstellen eines Übungsmodells für die Behandlung einer Person, welche an Störungen des zentralen Nervensystems leidet, wobei das Verfahren die folgenden Schritte aufweist:
- Identifizieren einer definierten Hirnregion der Person zur Beeinflussung der zugrunde liegenden Pathologie;
- Erfassen dreidimensionaler Bilddaten des Kopfes, d.h. von Gehirn und Schädel, der Person;
- Herstellen eines individualisierten rekonstruierten dreidimensionalen Kopfmodells auf Basis von dreidimensionalen Bilddaten des Kopfes; und
- Anordnen einer Vielzahl von Ultraschallsendern in einem Schädelknochenbereich des Kopfmodells, wobei die ausstrahlbaren Ultraschallwellen zu der definierten Hirnregion des Kopfmodells als gemeinsamem Fokus hin fokussiert werden. Definieren eines ausgeschnittenen Schädelknochenbereichs mit den darin angeordneten Ultraschallsendern als Modell für ein Implantat, wie es in Anspruch 1 definiert ist.

## Claims

1. An ultrasonic transmitter arrangement (1) with a plurality of ultrasonic transmitters (2) arranged in a distributed manner on the arrangement whose emittable ultrasonic waves are or can be focused toward a common focus (3), the arrangement (1) being embodied as a cranial bone implant (4) that can be implanted into a person's cranial bone (5) as a replacement for a portion thereof, and with the common focus (1) of the arrangement lying in a defined region (6) of the brain in the implanted state, or with it being possible for the common focus (3) to be guided there in the implanted state, **characterized in that** the arrangement is an individualized reconstructed implant that was manufactured on the basis of three-dimensional image data of a person's head, i.e. brain and cranial bone.

2. The ultrasonic transmitter arrangement (1) as set forth in claim 1, **characterized in that** the cranial bone implant (4) is embodied as a calotte and, in the implanted state, the ultrasound-emitting regions of the ultrasonic transmitters (2) are arranged on the surface of the calotte (4) facing toward the brain.

3. The ultrasonic transmitter arrangement (1) as set forth in claim 2, **characterized in that** the calotte (4) is modeled after the removed portion of the cranial bone (5).

4. The ultrasonic transmitter arrangement (1) as set forth in claim 2, **characterized in that** the calotte (4) is a spherical shell segment on which the ultrasonic transmitters (2) are arranged in a distributed manner, and the common focus of the ultrasonic transmitters (2) is the center point of the spherical shell segment.

5. The ultrasonic transmitter arrangement (1) as set forth in claim 4, **characterized in that** the outer edge (4a) of the calotte (4) is complementary to the inner edge (5a) of the opening in the cranium resulting from the removal of the portion of the cranial bone (5).

6. The ultrasonic transmitter arrangement as set forth in any one of claims 1 to 5, **characterized in that** the individualized reconstructed implant was manufactured by means of an additive process.

7. The ultrasonic transmitter arrangement as set forth in any one of the preceding claims, **characterized in that** a battery, particularly a rechargeable battery, is associated therewith.

8. The ultrasonic transmitter arrangement as set forth in any one of the preceding claims, **characterized in that** a generator for electrical energy, particularly a thermoelectric generator or a glucose biofuel cell, is associated therewith.

9. A method for manufacturing a training model for treating a person who suffers from disorders of the central nervous system, wherein the method has the following steps:
- identifying a defined region of the person's brain for influencing the underlying pathology;
- acquiring three-dimensional image data of the person's head, i.e. brain and cranium;
- preparing an individualized reconstructed three-dimensional head model on the basis of three-dimensional image data of the head;
- arranging a plurality of ultrasonic transmitters in a cranial bone region of the head model, with the emittable ultrasonic waves being focused, as a common focus, toward the defined region of the brain of the head model; and
- defining of a cut-out region of the cranial bone with the ultrasonic transmitters arranged therein as a model for an implant as set forth in claim 1.

## Revendications

1. Dispositif émetteur d'ultrasons (1) comportant plusieurs émetteurs d'ultrasons (2) disposés de manière répartie sur le dispositif et dont les ondes ultrasonores émises sont ou peuvent être focalisées en direction d'un foyer commun (3), le dispositif (1) se présentant sous la forme d'un implant osseux crânien (4) qui peut être implanté dans l'os crânien (5) d'une personne pour en remplacer une partie, le foyer commun (1) du dispositif se trouvant dans une région définie (6) du cerveau à l'état implanté, ou le foyer commun (3) pouvant être guidé à cet endroit à l'état implanté, **caractérisé en ce que** le dispositif est un implant reconstruit individualisé qui a été fabriqué sur la base de données d'images tridimensionnelles de la tête d'une personne, c'est-à-dire du cerveau et de l'os crânien.

2. Dispositif émetteur d'ultrasons (1) selon la revendication 1, **caractérisé en ce que** l'implant osseux crânien (4) se présente sous la forme d'une calotte et que, à l'état implanté, les zones émettrices d'ultrasons des émetteurs d'ultrasons (2) sont disposées sur la surface de la calotte (4) orientée vers le cerveau.

3. Dispositif émetteur d'ultrasons (1) selon la revendication 2, **caractérisé en ce que** la calotte (4) est modelée d'après la partie enlevée de l'os crânien (5).

4. Dispositif émetteur d'ultrasons (1) selon la revendication 2, **caractérisé en ce que** la calotte (4) est un segment de coque sphérique sur lequel les émetteurs d'ultrasons (2) sont disposés de manière répartie, et que le foyer commun des émetteurs d'ultrasons (2) est le point central de la calotte (4).

5. Dispositif émetteur d'ultrasons (1) selon la revendication 4, **caractérisé en ce que** le bord extérieur (4a) de la calotte (4) est complémentaire au bord intérieur (5a) de l'ouverture dans le crâne résultant de l'enlèvement de la partie de l'os crânien (5).

6. Dispositif émetteur d'ultrasons selon l'une quelconque des revendication 1 à 5, **caractérisé en ce que** l'implant reconstruit individualisé a été fabriqué au moyen d'un processus additif.

7. Dispositif émetteur d'ultrasons selon l'une quelconque des revendications précédentes, **caractérisé en ce qu'**il est associé à une batterie, notamment rechargeable.

8. Dispositif émetteur d'ultrasons selon l'une quelconque des revendications précédentes, **caractérisé en ce qu'**il est associé à un générateur d'énergie électrique, notamment un générateur thermoélectrique ou une pile à biocarburant (une bio-pile) à base de glucose.

9. Méthode de fabrication d'un modèle d'entraînement pour le traitement d'une personne souffrant de troubles du système nerveux central, dans laquelle la méthode comporte les étapes suivantes :
- identification d'une région définie du cerveau de la personne pour influencer la pathologie sous-jacente ;
- acquisition de données d'images tridimensionnelles de la tête de la personne, c'est-à-dire du cerveau et du crâne ;
- préparation d'un modèle de tête tridimensionnel reconstruit et individualisé sur la base des données d'images tridimensionnelles de la tête ;
- disposition d'une pluralité d'émetteurs d'ultrasons dans une région de l'os crânien du modèle de tête, les ondes ultrasonores susceptibles d'être émises étant focalisées en tant que foyer commun vers la région définie du cerveau du modèle de tête ; et
- définition d'une région découpée de l'os crânien avec les émetteurs d'ultrasons disposés à l'intérieur comme modèle pour un implant tel que défini dans la revendication 1.
